(19) Europäisches Patentamt
European Patent Office
Office européen des brevets

(11) Veröffentlichungsnummer: **0 111 234**
**B1**

# (12) EUROPÄISCHE PATENTSCHRIFT

(45) Veröffentlichungstag der Patentschrift: **08.10.86**

(51) Int. Cl.⁴: **A 01 N 43/64**, A 01 N 43/50, C 07 D 521/00

(21) Anmeldenummer: **83111884.9**

(22) Anmeldetag: **28.11.83**

## (54) Fungizide Mittel, deren Herstellung und Verwendung.

(30) Priorität: **09.12.82 DE 3245504**

(43) Veröffentlichungstag der Anmeldung: **20.06.84 Patentblatt 84/25**

(45) Bekanntmachung des Hinweises auf die Patenterteilung: **08.10.86 Patentblatt 86/41**

(84) Benannte Vertragsstaaten: **AT BE CH DE FR GB IT LI NL SE**

(56) Entgegenhaltungen:
**EP - A - 0 019 189**
**EP - A - 0 029 542**
**EP - A - 0 055 833**
**EP - A - 0 061 835**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen 1 (DE)**
Erfinder: **Reinecke, Paul, Dr., Lessingstrasse 11, D-5090 Leverkusen 3 (DE)**
Erfinder: **Rosslenbroich, Hans-Jürgen, Dr., Rembrandtstrasse 20, D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von substituierten Hydroxyalkylazolen zur Bekämpfung von phytopathogenen Pilzen.

Es ist bereits bekannt geworden, dass bestimmte Hydroxyalkylazol-Derivate gute fungizide Eigenschaften aufweisen (vergleiche DE-A 29 20 375, DE-A 29 20 374, DE-A 29 46 956 und DE-A 30 18 866). Die Wirkung dieser Verbindungen ist jedoch bei niedrigen Aufwandmengen und -konzentrationen nicht immer ganz befriedigend.

Ferner sind aus der EP-A 0061 835 Azolyl-Derivate mit fungiziden Eigenschaften bekannt. Es werden jedoch keine Verbindungen offenbart, in denen das Kohlenstoffatom, das eine Hydroxy-Gruppe und einen $-CH_2$-Azolyl-Rest trägt, gleichzeitig auch mit einem gegebenenfalls substituierten Phenyl-Rest und einer Gruppe der Formel

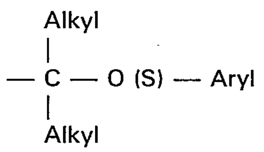

verbunden ist.

Schliesslich sind aus der EP-A 0090 993 substituierte Hydroxyalkylazole mit antimykotischer Wirksamkeit bekannt. Ein Einsatz der betreffenden Stoffe gegen phytopathogene Pilze wird jedoch nicht erwähnt.

Es wurde nun gefunden, dass sich die substituierten Hydroxyalkylazole der Formel

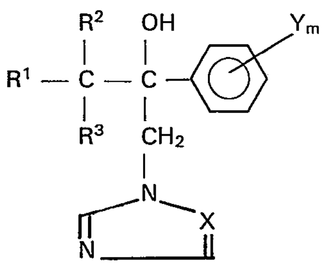

(I)

in welcher

$R^1$ für jeweils einfach bis zweifach, gleich oder verschieden substituiertes Phenyl, $-CH_2$-Phenyl, $-CH_2$-O-Phenyl, $-CH_2$-S-Phenyl, $-CH_2$-SO-Phenyl oder $-CH_2$-$SO_2$-Phenyl steht, wobei als Phenylsubstituenten die weiter unten angegebenen Bedeutungen von Y genannt seien;

oder

$R^1$ für jeweils einfach bis zweifach, gleich oder verschieden substituiertes -O-Phenyl, -S-Phenyl, -SO-Phenyl oder -$SO_2$-Phenyl steht, wobei als Phenylsubstituenten die weiter unten angegebenen Bedeutungen von Y genannt seien;

$R^2$ für geradkettiges oder verzweigtes Alkyl mit 1 bis 4 Kohlenstoffatomen steht;

$R^3$ für geradkettiges oder verzweigtes Alkyl mit 1 bis 4 Kohlenstoffatomen steht;

X für ein Stickstoffatom oder die CH-Gruppe steht;

Y für Halogen, Alkyl mit 1 bis 4 Kohlenstoffatomen, Cycloalkyl mit 5 bis 7 Kohlenstoffatomen, Alkoxy und Alkylthio mit 1 bis 4 Kohlenstoffatomen steht, für Halogenalkyl und Halogenalkoxy sowie Halogenalkylthio mit jeweils 1 bis 2 Kohlenstoff- und 1 bis 5 gleichen oder verschiedenen Halogenatomen steht, sowie für jeweils gegebenenfalls durch Halogen und Alkyl mit 1 bis 2 Kohlenstoffatomen substituiertes Phenyl, Phenoxy, Phenylalkyl sowie Phenylalkoxy mit 1 bis 2 Kohlenstoffatomen im Alkylteil bzw. im Alkoxyteil steht; und

m für die Zahlen 0, 1, 2 oder 3 steht,

bzw. deren Säureadditions-Salze und Metallsalz-Komplexe sehr gut zur Bekämpfung von Pilzen bei Pflanzen verwenden lassen.

Die Verbindungen der Formel (I) besitzen ein asymmetrisches Kohlenstoffatom und können deshalb in den beiden optischen Isomeren anfallen.

Überraschenderweise zeigen die erfindungsgemäss zu verwendenden substituierten Hydroxyalkylazole der Formel (I) eine bessere Wirksamkeit gegen phytopathogene Pilze als die aus dem Stand der Technik bereits bekannten Hydroxyalkylazolyl-Derivate, welche konstitutionell und wirkungsmässig naheliegende Verbindungen sind. Die erfindungsgemässe Verwendung der neuen Stoffe stellt somit eine Bereicherung der Technik dar.

Die erfindungsgemäss verwendbaren substituierten Hydroxyalkyl-azole besitzen ein Bauprinzip, das sich durch die Formel

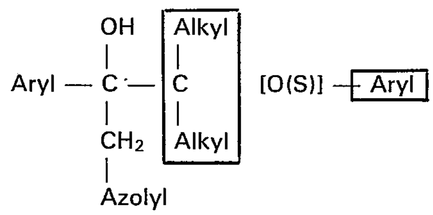

veranschaulichen lässt. Sie unterscheiden sich damit in charakteristischer Weise von den aus der EP-A 0061 835 bekannten Stoffen, denn dort werden — wie bereits erwähnt — keine Substanzen des oben aufgeführten Strukturtyps offenbart.

Erfindungsgemäss bevorzugt verwendbar sind diejenigen Verbindungen der Formel (I), in denen

$R^1$ für jeweils einfach bis zweifach, gleich oder verschieden substituiertes Phenyl, $-CH_2$-Phenyl, $-CH_2$-O-Phenyl, $-CH_2$-S-Phenyl, $-CH_2$-SO-Phenyl oder $-CH_2$-$SO_2$-Phenyl steht, wobei als Phenylsubstituenten die Bedeutungen von Y genannt seien;

$R^2$ für Methyl oder Ethyl steht;

$R^3$ für Methyl oder Ethyl steht;

X für ein Stickstoffatom oder die CH-Gruppe steht;

Y Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht; und

m für die Zahlen 0, 1 oder 2 steht.

Erfindungsgemäss bevorzugt verwendbar sind ausserdem diejenigen Verbindungen der Formel (I), in denen

$R^1$ für jeweils einfach bis zweifach, gleich oder verschieden substituiertes -O-Phenyl, -S-Phenyl, -SO-Phenyl oder $-SO_2$-Phenyl steht, wobei als Phenylsubstituenten die Bedeutungen von Y genannt seien;

$R^2$ für Methyl oder Ethyl steht;

$R^3$ für Methyl oder Ethyl steht;

X für ein Stickstoffatom oder die CH-Gruppe steht;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht, und

m für die Zahlen 0, 1 oder 2 steht.

Erfindungsgemäss bevorzugt verwendbare Verbindungen sind auch Additionsprodukte aus Säuren und diejenigen substituierten Hydroxyalkyl-azolen der Formel (I), in denen die Substituenten $R^1$, $R^2$, $R^3$, X und $Y_m$ die Bedeutungen haben, die bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Ausserdem erfindungsgemäss bevorzugt verwendbare Verbindungen sind Additionsprodukte aus Salzen von Metallen der II. bis IV. Haupt- und der I. und II. sowie IV. bis VIII. Nebengruppe und denjenigen substituierten Hydroxyalkyl-azolen der Formel (I), in denen die Substituenten $R^1$, $R^2$, $R^3$, X und $Y_m$ die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien ausser den bei den Herstellungsbeispielen genannten Verbindungen die folgenden Verbindungen der Formel (I) genannt (X steht sowohl für ein Stickstoffatom als auch die CH-Gruppe):

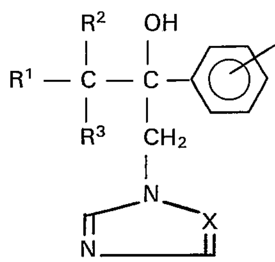

(I)

| $R^1$ | $R^2$ | $R^3$ | $Y_m$ |
|---|---|---|---|
| 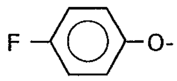 | $CH_3$ | $CH_3$ | 2-Cl |
| 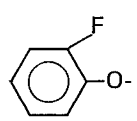 | $CH_3$ | $CH_3$ | 2-Cl |
| 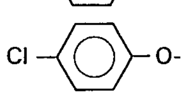 | $CH_3$ | $CH_3$ | 3-Cl |
| 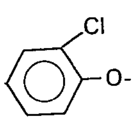 | $CH_3$ | $CH_3$ | 3,4-$Cl_2$ |
| 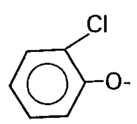 | $CH_3$ | $CH_3$ | 3-Cl |
| 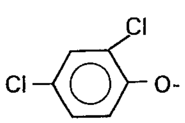 | $CH_3$ | $CH_3$ | 3-Cl |

| $R^1$ | $R^2$ | $R^3$ | $Y_m$ |
| --- | --- | --- | --- |
| 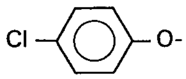 | $CH_3$ | $CH_3$ | 2,4-$Cl_2$ |
| 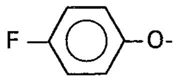 | $CH_3$ | $CH_3$ | 2,4-$Cl_2$ |
| 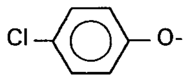 | $CH_3$ | $CH_3$ | 3,4-$Cl_2$ |
| 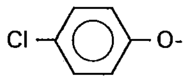 | $CH_3$ | $CH_3$ | 2-$CH_3$, 4-Cl |
| 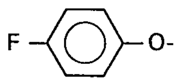 | $CH_3$ | $CH_3$ | 3,4-$Cl_2$ |
| 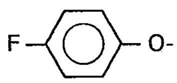 | $CH_3$ | $CH_3$ | 2-$CH_3$, 4-Cl |
| 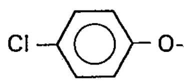 | $CH_3$ | $CH_3$ | 2-Cl |
| 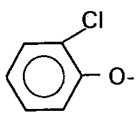 | $CH_3$ | $CH_3$ | 2-Cl |
| 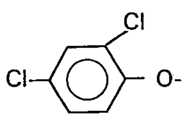 | $CH_3$ | $CH_3$ | 4-Cl |
| 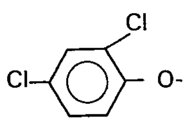 | $CH_3$ | $CH_3$ | 2,4-$Cl_2$ |
| 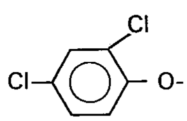 | $CH_3$ | $CH_3$ | 2-Cl |
| 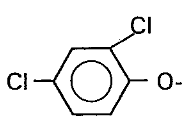 | $CH_3$ | $CH_3$ | 4-F |
| 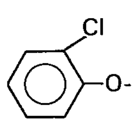 | $CH_3$ | $CH_3$ | 4-F |
| 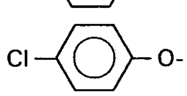 | $CH_3$ | $CH_3$ | 4 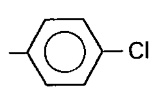 |
| 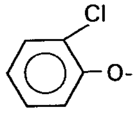 | $CH_3$ | $CH_3$ | 4 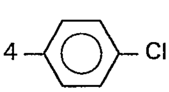 |

| $R^1$ | $R^2$ | $R^3$ | $Y_m$ |
|---|---|---|---|
| 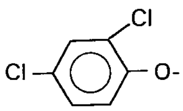 | $CH_3$ | $CH_3$ | 4 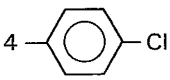 |
| 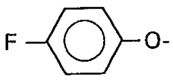 | $CH_3$ | $CH_3$ | 4 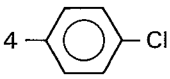 |
| 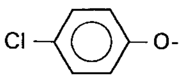 | $CH_3$ | $CH_3$ | 4-O 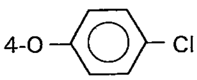 |
| 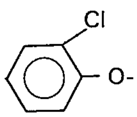 | $CH_3$ | $CH_3$ | 4-Cl |
| 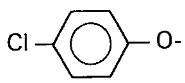 | $CH_3$ | $CH_3$ | 2-F |
| 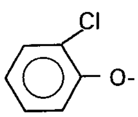 | $CH_3$ | $CH_3$ | 2-F |
| 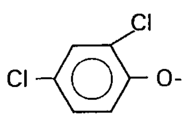 | $CH_3$ | $CH_3$ | 2-F |
| 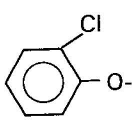 | $CH_3$ | $CH_3$ | 2,4-$Cl_2$ |
| 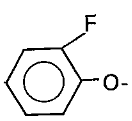 | $CH_3$ | $CH_3$ | 2,4-$Cl_2$ |
| 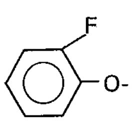 | $CH_3$ | $CH_3$ | 4-Cl |
| 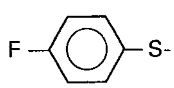 | $CH_3$ | $CH_3$ | 2-Cl |
| 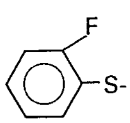 | $CH_3$ | $CH_3$ | 2-Cl |
| 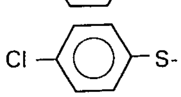 | $CH_3$ | $CH_3$ | 3-Cl |
| 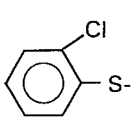 | $CH_3$ | $CH_3$ | 3,4-$Cl_2$ |

| $R^1$ | $R^2$ | $R^3$ | $Y_m$ |
|---|---|---|---|
| 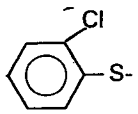 | $CH_3$ | $CH_3$ | 3-Cl |
| 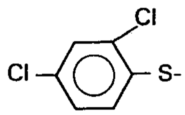 | $CH_3$ | $CH_3$ | 3-Cl |
| 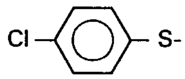 | $CH_3$ | $CH_3$ | 2,4-$Cl_2$ |
| 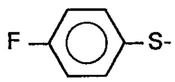 | $CH_3$ | $CH_3$ | 2,4-$Cl_2$ |
| 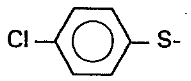 | $CH_3$ | $CH_3$ | 3,4-$Cl_2$ |
| 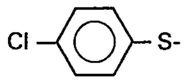 | $CH_3$ | $CH_3$ | 2-$CH_3$, 4-Cl |
| 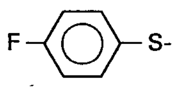 | $CH_3$ | $CH_3$ | 3,4-$Cl_2$ |
| 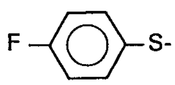 | $CH_3$ | $CH_3$ | 2-$CH_3$, 4-Cl |
| 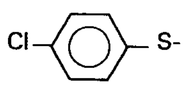 | $CH_3$ | $CH_3$ | 2-Cl |
| 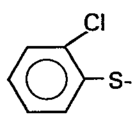 | $CH_3$ | $CH_3$ | 2-Cl |
| 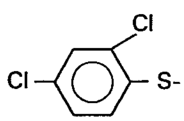 | $CH_3$ | $CH_3$ | 4-Cl |
| 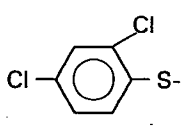 | $CH_3$ | $CH_3$ | 2,4-$Cl_2$ |
| 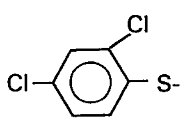 | $CH_3$ | $CH_3$ | 2-Cl |
| 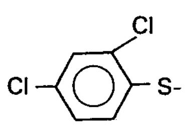 | $CH_3$ | $CH_3$ | 4-F |
| 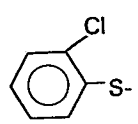 | $CH_3$ | $CH_3$ | 4-F |

| $R^1$ | $R^2$ | $R^3$ | $Y_m$ |
|---|---|---|---|
| 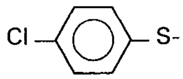 | $CH_3$ | $CH_3$ | 4 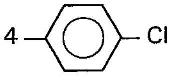 |
| 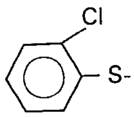 | $CH_3$ | $CH_3$ | 4 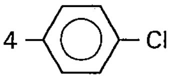 |
| 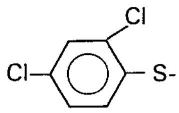 | $CH_3$ | $CH_3$ | 4 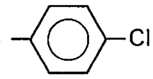 |
| 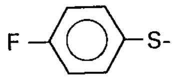 | $CH_3$ | $CH_3$ | 4 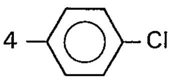 |
| 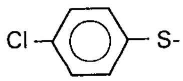 | $CH_3$ | $CH_3$ | 4-O 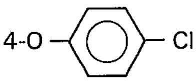 |
| 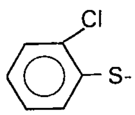 | $CH_3$ | $CH_3$ | 4-Cl |
| 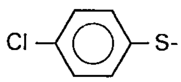 | $CH_3$ | $CH_3$ | 2-F |
| 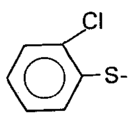 | $CH_3$ | $CH_3$ | 2-F |
| 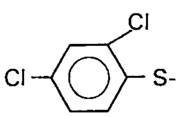 | $CH_3$ | $CH_3$ | 2-F |
| 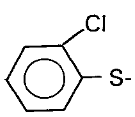 | $CH_3$ | $CH_3$ | 2,4-$Cl_2$ |
| 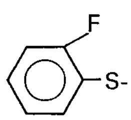 | $CH_3$ | $CH_3$ | 2,4-$Cl_2$ |
| 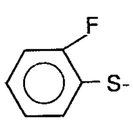 | $CH_3$ | $CH_3$ | 4-Cl |
| 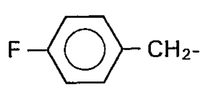 | $CH_3$ | $CH_3$ | 2-Cl |
| 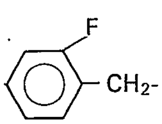 | $CH_3$ | $CH_3$ | 2-Cl |

| $R^1$ | $R^2$ | $R^3$ | $Y_m$ |
|---|---|---|---|
| 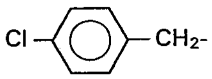 | $CH_3$ | $CH_3$ | 3-Cl |
| 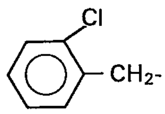 | $CH_3$ | $CH_3$ | 3,4-$Cl_2$ |
| 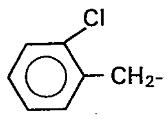 | $CH_3$ | $CH_3$ | 3-Cl |
| 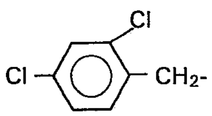 | $CH_3$ | $CH_3$ | 3-Cl |
| 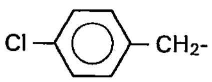 | $CH_3$ | $CH_3$ | 2,4-$Cl_2$ |
| 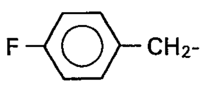 | $CH_3$ | $CH_3$ | 2,4-$Cl_2$ |
| 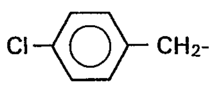 | $CH_3$ | $CH_3$ | 3,4-$Cl_2$ |
| 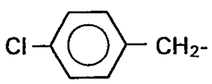 | $CH_3$ | $CH_3$ | 2-$CH_3$, 4-Cl |
| 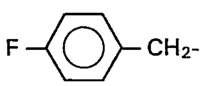 | $CH_3$ | $CH_3$ | 3,4-$Cl_2$ |
| 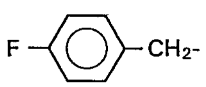 | $CH_3$ | $CH_3$ | 2-$CH_3$, 4-Cl |
| 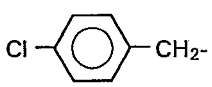 | $CH_3$ | $CH_3$ | 2-Cl |
| 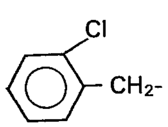 | $CH_3$ | $CH_3$ | 2-Cl |
| 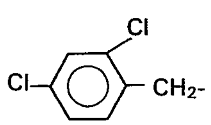 | $CH_3$ | $CH_3$ | 4-Cl |
| 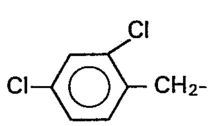 | $CH_3$ | $CH_3$ | 2,4-$Cl_2$ |
| 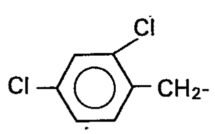 | $CH_3$ | $CH_3$ | 2-Cl |

| $R^1$ | $R^2$ | $R^3$ | $Y_m$ |
|---|---|---|---|
| 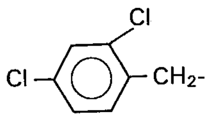 | $CH_3$ | $CH_3$ | 4-F |
| 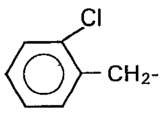 | $CH_3$ | $CH_3$ | 4-F |
| 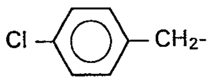 | $CH_3$ | $CH_3$ | 4 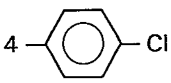 |
| 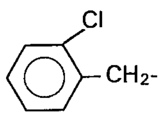 | $CH_3$ | $CH_3$ | 4 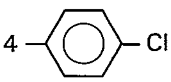 |
| 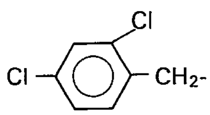 | $CH_3$ | $CH_3$ | 4 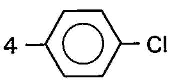 |
| 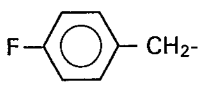 | $CH_3$ | $CH_3$ | 4 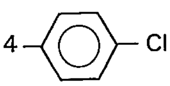 |
| 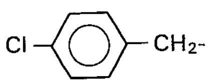 | $CH_3$ | $CH_3$ | 4-O 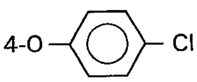 |
| 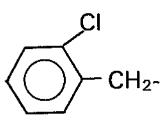 | $CH_3$ | $CH_3$ | 4-Cl |
| 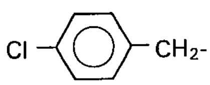 | $CH_3$ | $CH_3$ | 2-F |
| 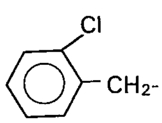 | $CH_3$ | $CH_3$ | 2-F |
| 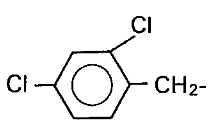 | $CH_3$ | $CH_3$ | 2-F |
| 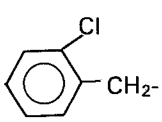 | $CH_3$ | $CH_3$ | 2,4-$Cl_2$ |
| 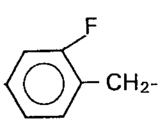 | $CH_3$ | $CH_3$ | 2,4-$Cl_2$ |
| 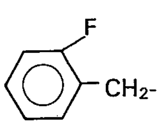 | $CH_3$ | $CH_3$ | 4-Cl |

| $R^1$ | $R^2$ | $R^3$ | $Y_m$ |
|---|---|---|---|
| 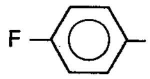 | $CH_3$ | $CH_3$ | 2-Cl |
| 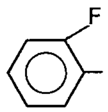 | $CH_3$ | $CH_3$ | 2-Cl |
| 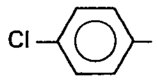 | $CH_3$ | $CH_3$ | 3-Cl |
| 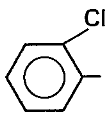 | $CH_3$ | $CH_3$ | 3,4-$Cl_2$ |
| 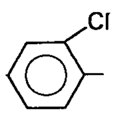 | $CH_3$ | $CH_3$ | 3-Cl |
| 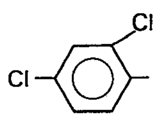 | $CH_3$ | $CH_3$ | 3-Cl |
| 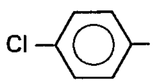 | $CH_3$ | $CH_3$ | 2,4-$Cl_2$ |
| 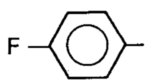 | $CH_3$ | $CH_3$ | 2,4-$Cl_2$ |
| 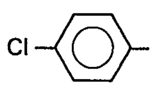 | $CH_3$ | $CH_3$ | 3,4-$Cl_2$ |
| 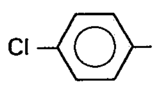 | $CH_3$ | $CH_3$ | 2-$CH_3$, 4-Cl |
| 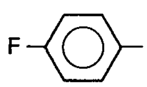 | $CH_3$ | $CH_3$ | 3,4-$Cl_2$ |
| 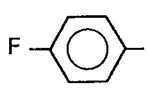 | $CH_3$ | $CH_3$ | 2-$CH_3$, 4-Cl |
| 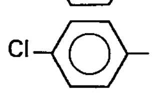 | $CH_3$ | $CH_3$ | 2-Cl |
| 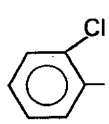 | $CH_3$ | $CH_3$ | 2-Cl |
| 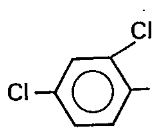 | $CH_3$ | $CH_3$ | 4-Cl |
| 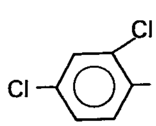 | $CH_3$ | $CH_3$ | 2,4-$Cl_2$ |

| $R^1$ | $R^2$ | $R^3$ | $Y_m$ |
|---|---|---|---|
| 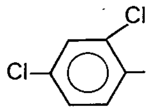 | $CH_3$ | $CH_3$ | 2-Cl |
| 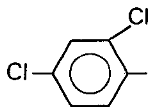 | $CH_3$ | $CH_3$ | 4-F |
| 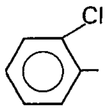 | $CH_3$ | $CH_3$ | 4-F |
| 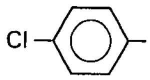 | $CH_3$ | $CH_3$ | 4 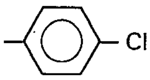 |
| 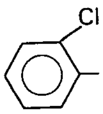 | $CH_3$ | $CH_3$ | 4 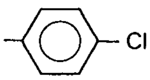 |
| 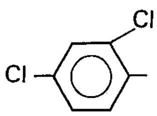 | $CH_3$ | $CH_3$ | 4 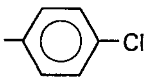 |
| 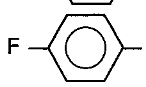 | $CH_3$ | $CH_3$ | 4 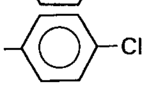 |
| 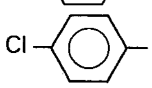 | $CH_3$ | $CH_3$ | 4-O 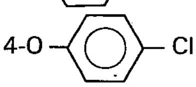 |
| 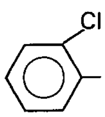 | $CH_3$ | $CH_3$ | 4-Cl |
| 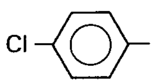 | $CH_3$ | $CH_3$ | 2-F |
| 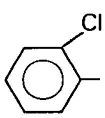 | $CH_3$ | $CH_3$ | 2-F |
| 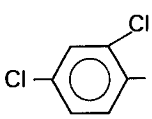 | $CH_3$ | $CH_3$ | 2-F |
| 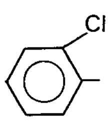 | $CH_3$ | $CH_3$ | $2,4-Cl_2$ |
| 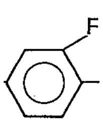 | $CH_3$ | $CH_3$ | $2,4-Cl_2$ |
| 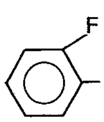 | $CH_3$ | $CH_3$ | 4-Cl |

Die erfindungsgemäss zu verwendenden Wirkstoffe sind Gegenstand der Patentanmeldung gemäss EP-A 0090993. Sie werden erhalten, indem man Oxirane der Formel

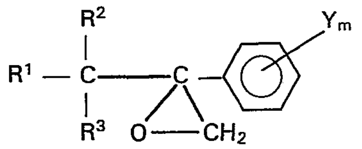

(II)

in welcher

$R^1$, $R^2$, $R^3$, Y und m die oben angegebene Bedeutung haben,

mit Azolen der Formel

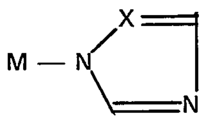

(III)

in welcher

X die oben angegebene Bedeutung hat und

M für Wasserstoff oder ein Alkalimetall, vorzugsweise Natrium oder Kalium, steht,

in Gegenwart eines inerten organischen Lösungsmittels, wie beispielsweise eines Alkohols, und gegebenenfalls in Gegenwart einer Base, wie beispielsweise eines Alkalimetallalkoholats, bei Temperaturen zwischen 60 und 150°C umsetzt. So erhaltene Hydroxyalkyl-azole der Formel

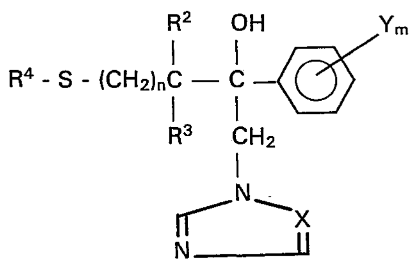

(Ia)

in welcher

$R^2$, $R^3$, X, Y und m die oben angegebene Bedeutung haben,

$R^4$ für gegebenenfalls ein- bis zweifach, gleich oder verschieden durch die für Y genannten Reste substituiertes Phenyl steht und

n für die Zahlen 0 oder 1 steht,

können nach bekannten Methoden in üblicher Weise oxidiert werden. Bei der Anwendung von 1 Mol Oxidationsmittel, wie m-Chlor-perbenzoesäure in Methylenchlorid oder Wasserstoffperoxid in Acetanhydrid bei Temperaturen zwischen —30 bis +30°C, entstehen vorzugsweise die erfindungsgemäss verwendbaren Verbindungen der Formel (I) mit der -SO-Gruppierung. Bei Überschuss an Oxidationsmittel und höheren Temperaturen (10 bis 80°C) entstehen vorzugsweise die erfindungsgemäss verwendbaren Verbindungen der Formel (I) mit der $-SO_2$-Gruppierung. Die Isolierung der Oxidationsprodukte erfolgt in üblicher Weise.

Die Oxirane der Formel (II) sind auch Gegenstand der Patentanmeldung gemäss EP-A 0090993. Sie werden erhalten, indem man Ketone der Formel

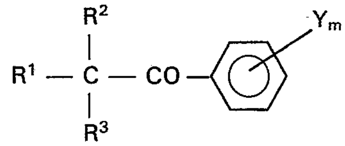

(IV)

in welcher

$R^1$, $R^2$, $R^3$, Y und m die oben angegebene Bedeutung haben,

entweder

α) mit Dimethyloxosulfonium-methylid der Formel

$$\overset{\delta+\delta}{(CH_3)_2SOCH_2} \quad (V)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu die Angaben in J. Am. Chem. Soc. 87, 1363-1364 [1965]), oder

β) mit Trimethylsulfonium-methylsulfat der Formel

$$\left[(CH_3)_3S^{(+)}\right] \; CH_3SO_4^{(-)} \quad (VI)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0 bis 60°C, vorzugsweise bei Raumtemperatur, umsetzt (vergleiche auch die Angaben in Heterocycles 8, 397 [1977]).

Die Säureadditionssalze der Verbindungen der Formel (I) können in ainfacher Weise nach üblichen Salzbildungsmethoden, durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, Isolieren und gegebenenfalls durch Umkristallisation reinigen.

Die erfindungsgemäss zu verwendenden Wirkstoffe weisen eine starke mikrobizide Wirkung auf und können zur Bekämpfung von unerwünschten Mikroorganismen bei Pflanzen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäss verwendbaren Wirkstoffe mit besonders gutem Erfolg zur Bekämpfung von Sphaerotheca-Arten, wie gegen den Erreger des echten Gurkenmehltaus (Sphaerotheca fuliginea), von Getreidekrankheiten, wie gegen den Erreger der Streifenkrankheit der Gerste (Drechslera graminea) oder gegen den Erreger der Braunfleckenkrankheit am Weizen (Leptosphaeria nodorum), sowie von Reiskrankheiten, wie Pyricularia oryzae und Pellicularia sasakii, eingesetzt werden.

In entsprechenden Aufwandmengen zeigen die erfindungsgemäss verwendbaren Wirkstoffe auch pflanzenwachstumsregulierende und selektiv herbizide Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäss verwendbaren Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

*Herstellungsbeispiele*

*Beispiel 1*

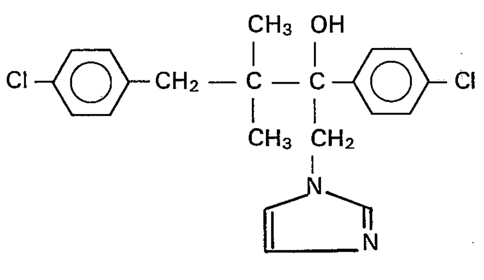

Zu einer Lösung von 7,7 g (0,108 Mol) Imidazol-Natrium in 60 ml n-Propanol werden bei Rückflusstemperatur 30 g (0,0935 Mol) 2-(4-Chlorphenyl)-2-(4-chlorphenyl-tert.-butyl)-oxiran in 40 ml n-Propanol getropft. Man lässt das Reaktionsgemisch 48 Stunden unter Rückfluss nachrühren, kühlt ab, versetzt mit Wasser und extrahiert mit Methylenchlorid. Die organische Phase wird über Natriumsulfat getrocknet und im Vakuum eingeengt. Der ölige Rückstand wird in Diisopropylether verrührt. Der entstehende kristalline Niederschlag wird abgesaugt und getrocknet. Man erhält 12,7 g (35% der Theorie) 2,4-Bis(4-chlorphenyl)-3,3-dimethyl-1-(imidazol-1-yl)-2-butanol vom Schmelzpunkt 174°C.

*Herstellung des Ausgangsproduktes*

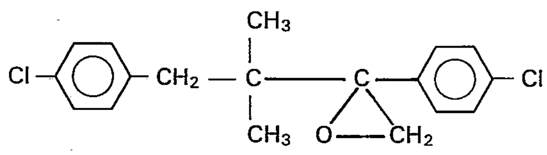

Eine Lösung von 59,2 g (0,47 Mol) Dimethylsulfat und 32 g (0,517 Mol) Dimethylsulfid in 270 ml Acetonitril lässt man 5 Tage bei Raumtemperatur rühren. Innerhalb von ca. 2 Stunden lässt man dann bei 20 bis 25°C eine Lösung von 81,5 g (0,2655 Mol) 4-Chlorphenyl-(4-chlorphenyl-tert.-butyl)-keton in 80 ml Acetonitril zutropfen. Bei gleicher Temperatur werden 28,7 g (0,53 Mol) Natriummethylat zugegeben. Man lässt das gesamte Reaktionsgemisch 12 Stunden nachrühren und engt danach im Vakuum ein. Der Rückstand wird mit einem Gemisch aus 200 ml Essigester und 150 ml Wasser über Nacht verrührt. Die organische Phase wird abgetrennt, über Natriumsulfat getrocknet und im Vakuum eingeengt. Man erhält 72,6 g (85,2% der Theorie) rohes 2-(4-Chlorphenyl)-2-(4-chlorphenyl-tert.-butyl)-oxiran, das direkt weiter umgesetzt wird.

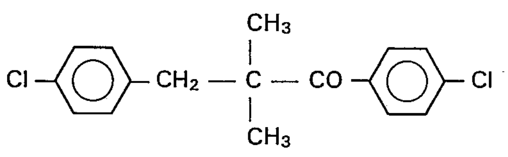

85 g (0,466 Mol) 4-Chlorphenyl-isopropyl-keton, 31,3 g (0,56 Mol) Kaliumhydroxid und 5 g Tetrabutylammoniumbromid in 120 ml Toluol werden auf Rückflusstemperatur erhitzt und tropfenweise mit einer Lösung von 75 g (0,466 Mol) 4-Chlorbenzylchlorid in 60 ml Toluol versetzt. Man lässt das Reaktionsgemisch 12 Stunden unter Rückfluss nachrühren, kühlt ab, wäscht mit Wasser, trocknet die organische Phase über Natriumsulfat und engt im Vakuum ein. Man erhält 81,5 g (60% der Theorie) 4-Chlorphenyl-(4-chlorphenyl-tert.-butyl)-keton vom Brechungsindex $n_D^{20} = 1,5711$.

*Beispiel 2*

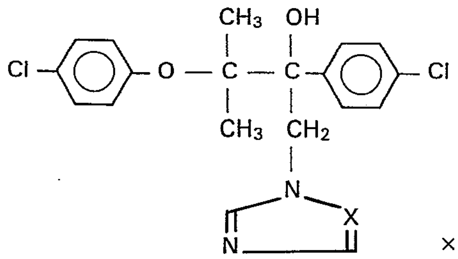

× HCl

Eine Lösung von 30 g (0,093 Mol) 2-(4-Chlorphenyl)-2-[2-(p-Chlorphenoxy)-prop-2-yl]-oxiran in 40 ml n-Propanol wird bei Raumtemperatur zu einer Lösung von 7,6 g (0,107 Mol) 1,2,4-Triazol-Natrium in 60 ml n-Propanol getropft. Man lässt das Reaktionsgemisch 48 Stunden bei Rückflusstemperatur nachrühren, kühlt ab, versetzt mit Wasser und extrahiert mit Methylenchlorid. Die organische Phase wird über Natriumsulfat getrocknet und im Vakuum eingeengt. Der ölige Rückstand wird säulenchromatographisch gereinigt. Man erhält 6,7 g (18,4% der Theorie) 3-(4-Chlorphenoxy)-2-(4-chlorphenyl)-3-methyl-1-(1,2,4-triazol-1-yl)-2-butanol. Diese werden bei Raumtemperatur mit 20 ml gesättigter Chlorwasserstoff/Ether-Lösung verrührt. Der ausfallende Niederschlag wird abgesaugt, mit wenig Ether nachgewaschen und im Vakuum bei 40°C getrocknet. Man erhält 6,5 g (89% der Theorie bezogen auf eingesetzte Base) 3-(4-Chlorphenoxy)-2-(4-chlorphenyl)-3-methyl-1-(1,2,4-triazol-1-yl)-2-butanol-hydrochlorid vom Schmelzpunkt 135°C.

*Herstellung des Ausgangsproduktes*

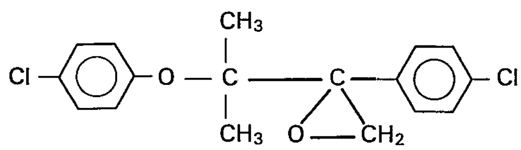

Eine Lösung von 59,2 g (0,47 Mol) Dimethylsulfat und 32 g (0,517 Mol) Dimethylsulfid in 270 ml Acetonitril lässt man 5 Tage bei Raumtemperatur rühren. Innerhalb von ca. 2 Stunden lässt man dann bei 20 bis 25°C eine Lösung von 87 g 4-Chlorphenyl-[2-(p-chlorphenoxy)-prop-2-yl]-keton in 80 ml Acetonitril zutropfen. Bei gleicher Temperatur trägt man 28,7 g (0,53 Mol) Natriummethylat ein, lässt 12 Stunden nachrühren und engt anschliessend ein. Der Rückstand wird mit einem Gemisch aus 200 ml Essigester und 150 ml Wasser über Nacht verrührt. Die organische Phase wird abgetrennt, über Natriumsulfat getrocknet und im Vakuum eingeengt. Man erhält 49 g (76% der Theorie) rohes 2-(4-Chlorphenyl)-2-[2-(p-chlorphenoxy)-prop-2-yl]-oxiran, das direkt weiter umgesetzt wird.

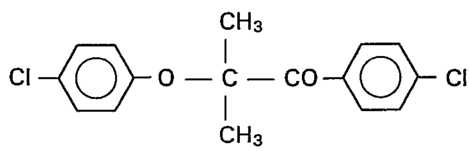

52 g (0,3982 Mol) p-Chlorphenol und 55 g (0,3982 Mol) Kaliumcarbonat in 400 ml Toluol werden 2 Stunden unter Rückfluss am Wasserabscheider erhitzt. Man kühlt auf 40°C ab und versetzt tropfenweise mit einer Lösung von 2-Brom-prop-2-yl--(4-chlorphenyl)-keton in 170 ml Toluol. Dieses Reaktionsgemisch lässt man 5 Stunden bei 100°C nachrühren, kühlt anschliessend ab, versetzt mit Wasser und trennt die organische Phase ab. Diese wird mit verdünnter Natronlauge sowie Wasser gewaschen, über Natriumsulfat getrocknet und eingeengt. Man erhält 87 g (85% der Theorie) rohes 4--Chlorphenyl-[2-(p-chlorphenoxy)-prop-2-yl]-keton, das direkt weiter umgesetzt wird.

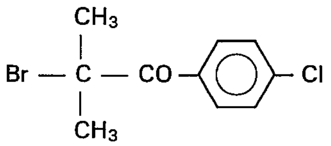

Zu 65,5 g (0,36 Mol) 4-Chlorphenyl-isopropyl--keton in 200 ml Chloroform gibt man 1 ml Bromwasserstoff/Eisessig zu und lässt dann bei 30°C 57,5 g (0,36 Mol) Brom zutropfen. Man lässt 30 Minuten bei Raumtemperatur nachrühren und engt danach im Vakuum ein. Man erhält 86,6 g (92% der Theorie) rohes 2-Brom-prop-2-yl-(4-chlorphenyl)--keton, das direkt weiter umgesetzt wird.

In entsprechender Weise und gemäss dem beschriebenen Verfahren werden die folgenden Verbindungen der allgemeinen Formel

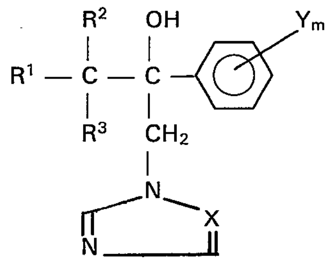 (I)

erhalten:

| Bsp. Nr. | $R^1$ | $R^2$ | $R^3$ | X | $Y_m$ | Schmelzpunkt (°C) |
|---|---|---|---|---|---|---|
| 3 | 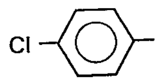 | $CH_3$ | $CH_3$ | CH | 4-Cl | 138 |
| 4 | 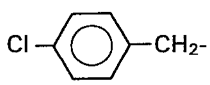 | $CH_3$ | $CH_3$ | N | 4-Cl | 137 |
| 5 | 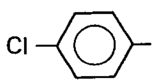 | $CH_3$ | $CH_3$ | N | 4-Cl | 109 |
| 6 | 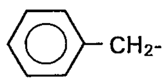 | $CH_3$ | $CH_3$ | N | 4-F | 120 |
| 7 | 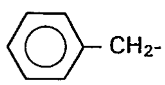 | $CH_3$ | $CH_3$ | CH | 4-F | 118 - 120 |
| 8 | 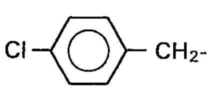 | $CH_3$ | $CH_3$ | CH | 4-F | 165 - 167 |
| 9 | 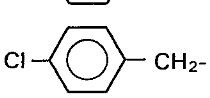 | $CH_3$ | $CH_3$ | N | 4-F | 124 |
| 10 | 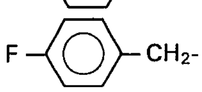 | $CH_3$ | $CH_3$ | CH | 4-Cl | 126 |
| 11 | 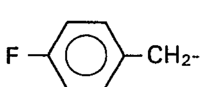 | $CH_3$ | $CH_3$ | N | 4-Cl | 112 |
| 12 | 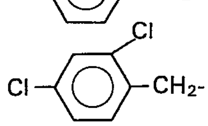 | $CH_3$ | $CH_3$ | CH | 4-F | 93 |
| 13 | 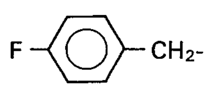 | $CH_3$ | $CH_3$ | CH | — | 164 - 65 |
| 14 | 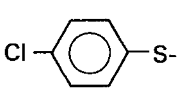 | $CH_3$ | $CH_3$ | N | 4-Cl | 74 - 76 |

| Bsp. Nr. | $R^1$ | $R^2$ | $R^3$ | X | $Y_m$ | Schmelz-punkt (°C) |
|---|---|---|---|---|---|---|
| 15 | 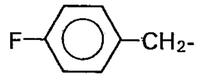 | $CH_3$ | $CH_3$ | N | — | 102 - 04 |
| 16 | 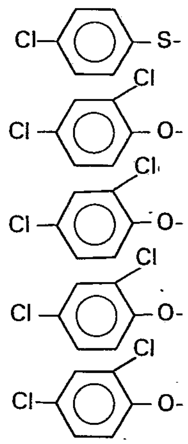 | $CH_3$ | $CH_3$ | CH | 4-Cl | 94 |
| 17 | | $CH_3$ | $CH_3$ | CH | 4-Cl | 80 - 82 |
| 18 | | $CH_3$ | $CH_3$ | N | 4-Cl | 96 - 98 |
| 19 | | $CH_3$ | $CH_3$ | N | 4-Cl | 114 - 16 |
| 20 | | $CH_3$ | $CH_3$ | CH | 4-Cl | 194 |
| 21 | 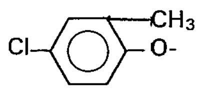 | $CH_3$ | $CH_3$ | N | 4-Cl | 117 |
| 22 | 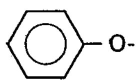 | $CH_3$ | $CH_3$ | N | 4-Cl | 82 |
| 23 | 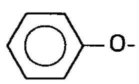 | $CH_3$ | $CH_3$ | CH | 4-Cl | 188 |
| 24 | 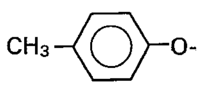 | $CH_3$ | $CH_3$ | N | 4-Cl | 62 |
| 25 | 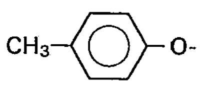 | $CH_3$ | $CH_3$ | CH | 4-Cl | 106 |
| 26 | 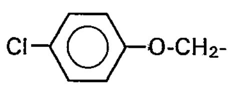 | $CH_3$ | $CH_3$ | N | 4-Cl | 104 - 06 |
| 27 | 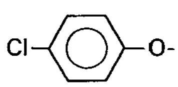 | $C_2H_5$ | $CH_3$ | N | 4-Cl | 182 - 84 (× HCl) |
| 28 | 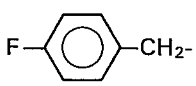 | $CH_3$ | $CH_3$ | N | 4-F | 118 |
| 29 | 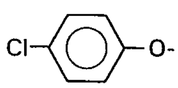 | $CH_3$ | $CH_3$ | N | 4-F | 82 |
| 30 | 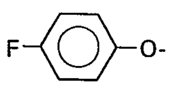 | $CH_3$ | $CH_3$ | N | 4-F | 78 |
| 31 | 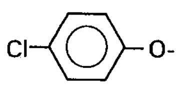 | $CH_3$ | $CH_3$ | CH | 4-F | 154 |
| 32 | 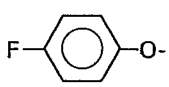 | $CH_3$ | $CH_3$ | CH | 4-F | 178 |
| 33 | 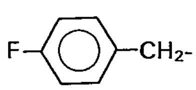 | $CH_3$ | $CH_3$ | CH | 4-F | 50 |
| 34 | 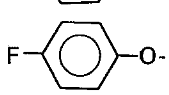 | $C_2H_5$ | $CH_3$ | N | 4-F | 114 |

| Bsp. Nr. | $R^1$ | $R^2$ | $R^3$ | X | $Y_m$ | Schmelz-punkt (°C) |
|---|---|---|---|---|---|---|
| 35 | 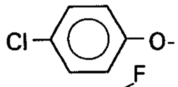 | $C_2H_5$ | $CH_3$ | N | 4-F | 66 |
| 36 | 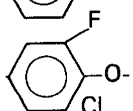 | $CH_3$ | $CH_3$ | N | 4-F | 120 |
| 37 | 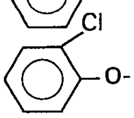 | $CH_3$ | $CH_3$ | N | 4-F | 147 |

*Verwendungsbeispiele*

In den nachfolgenden Beispielen werden die nachstehend angegebenen Verbindungen als Vergleichssubstanzen eingesetzt.

(A) 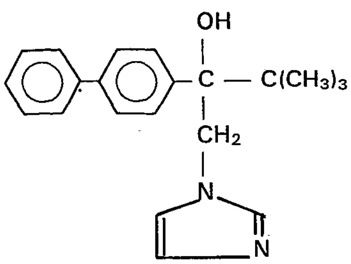

(B) 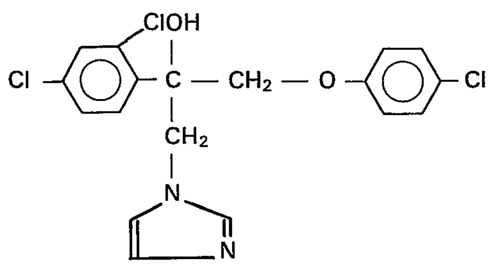

(C) 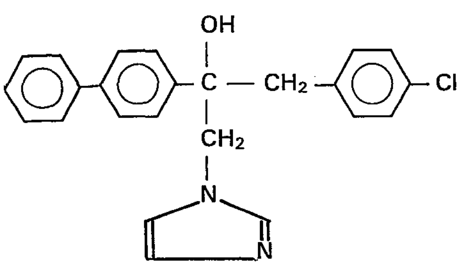

(D) 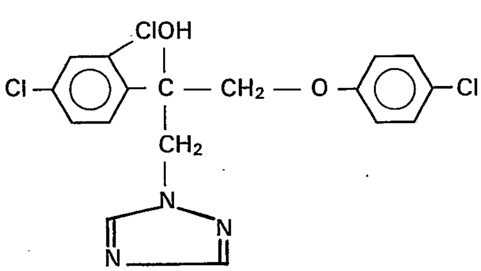

(E) 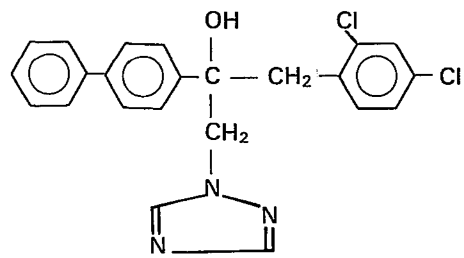

*Beispiel A*

Sphaerotheca-Test (Gurke) / protektiv

Lösungsmittel: 4,4 Gewichtsteile Aceton
Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Spaerotheca fuliginea bestäubt.

Die Pflanzen werden anschliessend bei 23 bis 24°C und bei einer relativen Luftfeuchtigkeit von ca. 75% im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 1, 4, 2, 5, 9, 6, 7, 11, 14 und 18.

*Tabelle A*

Sphaerotheca-Test (Gurke) / protektiv

| Wirkstoff | Befall in % bei einer Wirkstoffkonzentration von 50ppm | 5ppm |
|---|---|---|
| 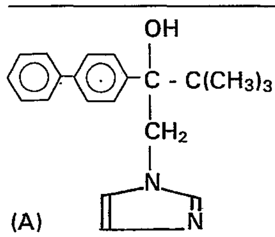 (A) (bekannt) | 100 | |

*Tabelle A (Fortsetzung)*
Sphaerotheca-Test (Gurke) / protektiv

| Wirkstoff | Befall in % bei einer Wirkstoffkonzentration von | |
|---|---|---|
| | 50ppm | 5ppm |
| 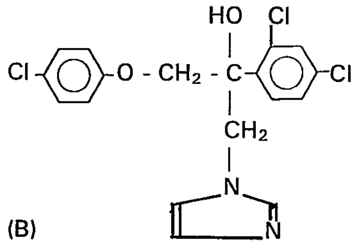 (B) (bekannt) | | 25 |
| 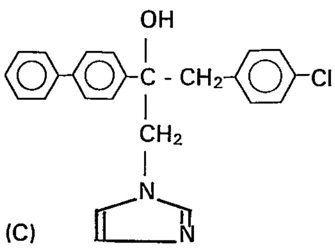 (C) (bekannt) | 100 | |
| 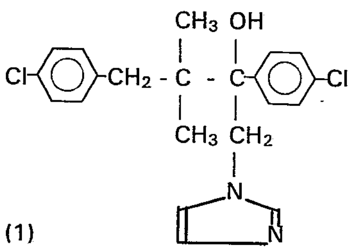 (1) | | 20 |
| 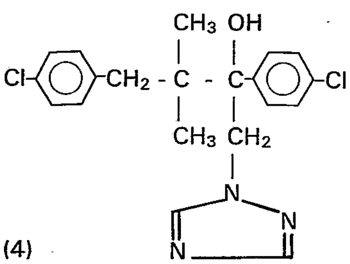 (4) | | 7 |
| 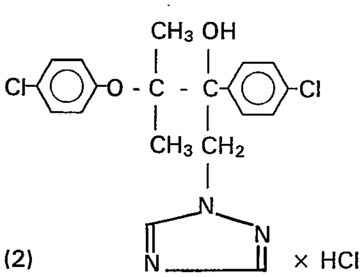 (2) × HCl | | 0 |

*Tabelle A (Fortsetzung)*
Sphaerotheca-Test (Gurke) / protektiv

| Wirkstoff | Befall in % bei einer Wirkstoffkonzentration von | |
|---|---|---|
| | 50ppm | 5ppm |
| 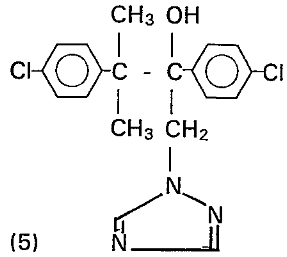 (5) | | 0 |
| 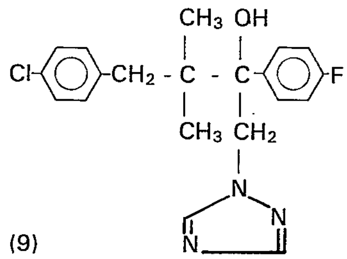 (9) | | 13 |
| 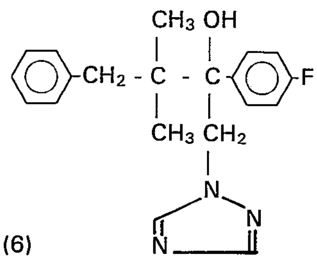 (6) | | 10 |
| 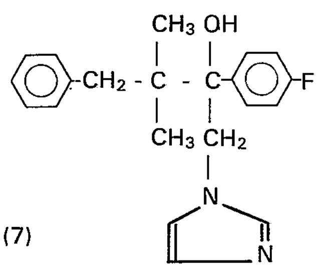 (7) | | 7 |
| 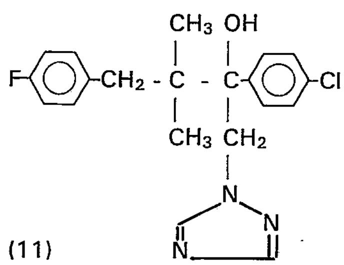 (11) | | 10 |

*Tabelle A (Fortsetzung)*

Sphaerotheca-Test (Gurke) / protektiv

| Wirkstoff | Befall in % bei einer Wirkstoffkonzentration von 50ppm | 5ppm |
|---|---|---|
| 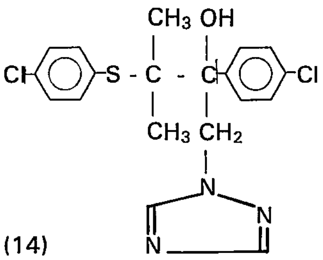 (14) | 10 | |
| 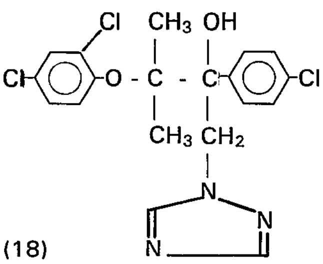 (18) | 10 | |

*Beispiel B*

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmässige Verteilung auf der Saatgutoberfläche gewährleistet.

Zur Beziehung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschliessend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäss folgender Herstellungsbeispiele: 6 und 7.

*Tabelle B*

Drechslera graminea-Test (Gerste) / Saatgutbehandlung (syn. Helminthosporium gramineum)

| Wirkstoff | Wirkstoffaufwandmenge in mg/kg Saatgut | kranke Pflanzen in % der insgesamt aufgelaufenen Pflanzen |
|---|---|---|
| ungebeizt | — | 25,5 |
| 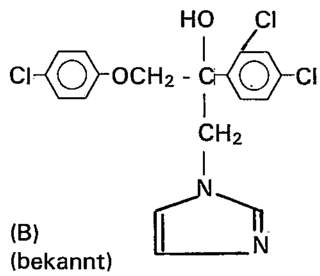 (B) (bekannt) | 500 | 23,9 |
| 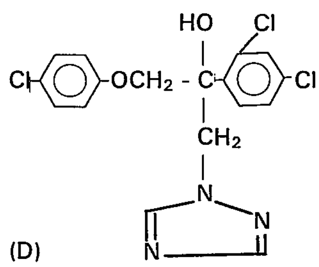 (D) (bekannt) | 500 | 20,7 |
| 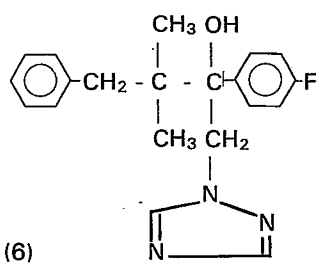 (6) | 500 | 3,7 |
| 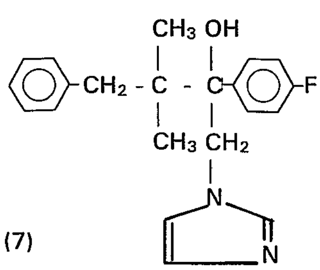 (7) | 500 | 4,9 |

*Beispiel C*

Leptosphaeria nodorum-Test (Weizen) / protektiv

| | | |
|---|---|---|
| Lösungsmittel: | 100 | Gewichtsteile Dimethylformamid |
| Emulgator: | 0,25 | Gewichtsteile Alkylarylpolyglykolether |

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden im Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 1, 4, 5 und 3.

*Tabelle C*

Leptosphaeria nodorum-Test (Weizen) / protektiv

| Wirkstoff | Wirkstoffkonzentration in der Spritzbrühe in Gew.-% | Krankheitsbefall in % der unbehandelten Kontrolle |
|---|---|---|
| 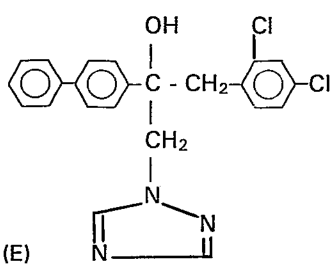 (E) (bekannt) | 0,025 | 78,5 |
| 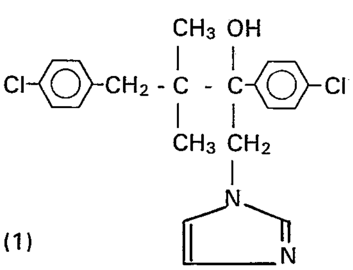 (1) | 0,025 | 16,2 |

*Tabelle C (Fortsetzung)*

Leptosphaeria nodorum-Test (Weizen) / protektiv

| Wirkstoff | Wirkstoffkonzentration in der Spritzbrühe in Gew.-% | Krankheitsbefall in % der unbehandelten Kontrolle |
|---|---|---|
| 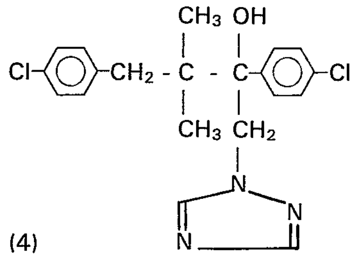 (4) | 0,025 | 16,2 |
| 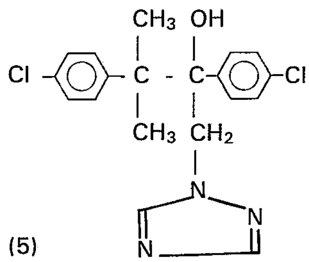 (5) | 0,025 | 37,0 |
| 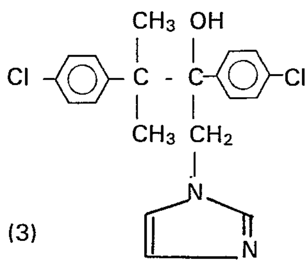 (3) | 0,025 | 37,0 |

*Beispiel D*

Pyricularia-Test (Reis) / protektiv

| | | |
|---|---|---|
| Lösungsmittel: | 12,5 | Gewichtsteile Aceton |
| Emulgator: | 0,3 | Gewichtsteile Alkylarylpolyglykolether |

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: 7, 15, 18 und 19.

*Tabelle D*

Pyricularia-Test (Reis) / protektiv

| Wirkstoff | Wirkstoffkonzentration in % | Krankheitsbefall in % der unbehandelten Kontrolle |
|---|---|---|
| 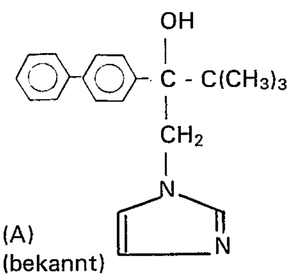 (A) (bekannt) | 0,025 | 100 |
| 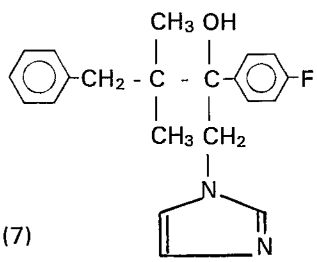 (7) | 0,025 | 25 |
| 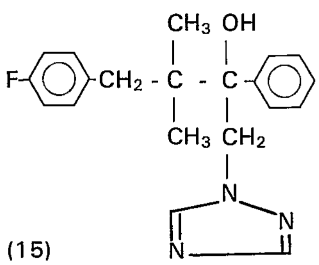 (15) | 0,025 | 25 |
| 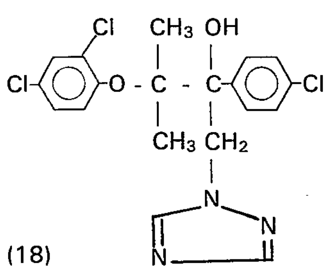 (18) | 0,025 | 25 |

*Tabelle D (Fortsetzung)*

Pyricularia-Test (Reis) / protektiv

| Wirkstoff | Wirkstoffkonzentration in % | Krankheitsbefall in % der unbehandelten Kontrolle |
|---|---|---|
| 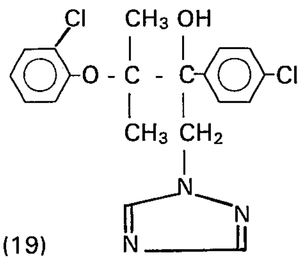 (19) | 0,025 | 30 |

**Patentansprüche**

1. Verwendung von substituierten Hydroxyalkylazolen der Formel

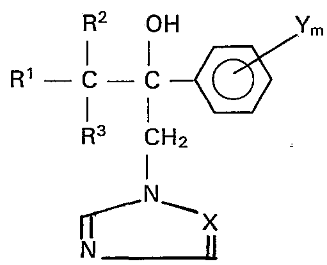

(I)

in welcher

$R^1$ für jeweils einfach bis zweifach, gleich oder verschieden substituiertes Phenyl, $-CH_2$-Phenyl, $-CH_2$-O-Phenyl, $-CH_2$-S-Phenyl, $-CH_2$-SO-Phenyl oder $-CH_2-SO_2$-Phenyl steht, wobei als Phenylsubstituenten die weiter unten angegebenen Bedeutungen von Y genannt seien;

oder

$R^1$ für jeweils einfach bis zweifach, gleich oder verschieden substituiertes -O-Phenyl, -S-Phenyl, -SO-Phenyl oder $-SO_2$-Phenyl steht, wobei als Phenylsubstituenten die weiter unten angegebenen Bedeutungen von Y genannt seien;

$R^2$ für geradkettiges oder verzweigtes Alkyl mit 1 bis 4 Kohlenstoffatomen steht;

$R^3$ für geradkettiges oder verzweigtes Alkyl mit 1 bis 4 Kohlenstoffatomen steht;

X für ein Stickstoffatom oder die CH-Gruppe steht;

Y für Halogen, Alkyl mit 1 bis 4 Kohlenstoffatomen, Cycloalkyl mit 5 bis 7 Kohlenstoffatomen, Alkoxy und Alkylthio mit 1 bis 4 Kohlenstoffatomen steht, für Halogenalkyl und Halogenalkoxy sowie Halogenalkylthio mit jeweils 1 bis 2 Kohlenstoff- und 1 bis 5 gleichen oder verschiedenen Halogenatomen

steht, sowie für jeweils gegebenenfalls durch Halogen und Alkyl mit 1 bis 2 Kohlenstoffatomen substituiertes Phenyl, Phenoxy, Phenylalkyl sowie Phenylalkoxy mit 1 bis 2 Kohlenstoffatomen im Alkylteil bzw. im Alkoxyteil steht; und

m für die Zahlen 0, 1, 2 oder 3 steht,

bzw. deren Säureadditions-Salzen und Metallsalz-Komplexen zur Bekämpfung von Pilzen bei Pflanzen.

2. Verwendung gemäss Anspruch 1, dadurch gekennzeichnet, dass in der Formel (I)

$R^1$ für jeweils einfach bis zweifach, gleich oder verschieden substituiertes Phenyl, $-CH_2$-Phenyl, $-CH_2-O$-Phenyl, $-CH_2-S$-Phenyl, $-CH_2-SO$-Phenyl oder $-CH_2-SO_2$-Phenyl steht, wobei als Phenylsubstituenten die weiter unten erwähnten Bedeutungen von Y genannt seien;

$R^2$ für Methyl oder Ethyl steht;

$R^3$ für Methyl oder Ethyl steht;

X für ein Stickstoffatom oder die CH-Gruppe steht;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht; und

m für die Zahlen 0, 1 oder 2 steht.

3. Verwendung gemäss Anspruch 1, dadurch gekennzeichnet, dass in der Formel (I)

$R^1$ für jeweils einfach bis zweifach, gleich oder verschieden substituiertes -O-Phenyl, -S-Phenyl, -SO-Phenyl oder $-SO_2$-Phenyl steht, wobei als Phenylsubstituenten die weiter unten erwähnten Bedeutungen von Y genannt seien;

$R^2$ für Methyl oder Ethyl steht;

$R^3$ für Methyl oder Ethyl steht;

X für ein Stickstoffatom oder die CH-Gruppe steht;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht, und

m für die Zahlen 0, 1 oder 2 steht.

**Claims**

1. Use of substituted hydroxyalkyl-azoles of the formula

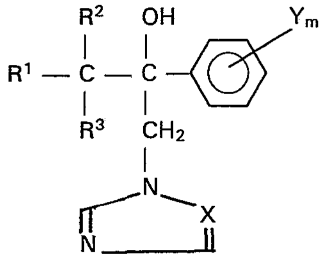

(I)

in which

$R^1$ represents phenyl, $-CH_2$-phenyl, $-CH_2-O$-phenyl, $-CH_2-S$-phenyl, $-CH_2-SO$-phenyl or $-CH_2-SO_2$-phenyl, in each case mono- to disubstituted by identical or different substituents, the phenyl substituents which may be mentioned being the meanings of Y given further below;

or

$R^1$ represents -O-phenyl, -S-phenyl, -SO-phenyl or $-SO_2$-phenyl, in each case mono- to disubstituted by identical or different substituents, the phenyl substituents which may be mentioned being the meanings of Y given further below;

$R^2$ represents straight-chain or branched alkyl with 1 to 4 carbon atoms;

$R^3$ represents straight-chain or branched alkyl with 1 to 4 carbon atoms;

X represents a nitrogen atom or the CH group;

Y represents halogen, alkyl with 1 to 4 carbon atoms, cycloalkyl with 5 to 7 carbon atoms, alkoxy and alkylthio with 1 to 4 carbon atoms, halogenoalkyl and halogenoalkoxy and halogenoalkylthio with in each case 1 to 2 carbon and 1 to 5 identical or different halogen atoms, and phenyl, phenoxy, phenylalkyl and phenylalkoxy with 1 to 2 carbon atoms in the alkyl part or in the alkoxy part, each of which is optionally substituted by halogen and alkyl with 1 to 2 carbon atoms; and

m represents the numbers 0, 1, 2 or 3,

or their acid addition salts and metal salt complexes for combating fungi in plants.

2. Use according to Claim 1, characterised in that in the formula (I)

$R^1$ represents phenyl, $-CH_2$-phenyl, $-CH_2-O$-phenyl, $-CH_2-S$-phenyl, $-CH_2-SO$-phenyl or $-CH_2-SO_2$-phenyl, in each case mono- to disubstituted by identical or different substituents, the phenyl substituents which may be mentioned being the meanings of Y mentioned further below;

$R^2$ represents methyl or ethyl;

$R^3$ represents methyl or ethyl;

X represents a nitrogen atom or the CH group;

Y represents fluorine, chlorine, bromine, methyl, isopropyl, tert.-butyl, cyclohexyl, methoxy, methylthio, trifluoromethyl, trifluoromethoxy, trifluoromethylthio and phenyl, phenoxy, benzyl or benzyloxy, optionally substituted by fluorine, chlorine or methyl;

and

m represents the numbers 0, 1 or 2.

3. Use according to Claim 1, characterised in that in the formula (I)

$R^1$ represents -O-phenyl, -S-phenyl, -SO-phenyl or $-SO_2$-phenyl, in each case mono- to disubstituted by identical or different substituents, the phenyl substituents which may be mentioned being the meanings of Y mentioned further below;

$R^2$ represents methyl or ethyl;

$R^3$ represents methyl or ethyl;

X represents a nitrogen atom or the CH group;

Y represents fluorine, chlorine, bromine, methyl, isopropyl, tert.-butyl, cyclohexyl, methoxy, methylthio, trifluoromethyl, trifluoromethoxy, trifluoromethylthio and phenyl, phenoxy, benzyl or benzyloxy, optionally substituted by fluorine, chlorine or methyl, and

m represents the numbers 0, 1 or 2.

**Revendications**

1. Utilisation d'hydroxyalcoylazols substitués de formule:

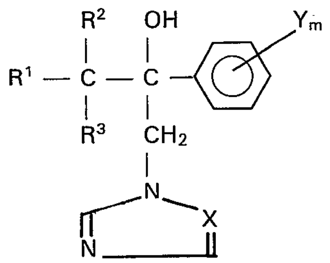

(I)

dans laquelle

$R^1$ représente un phényle, -$CH_2$-phényle, -$CH_2$-O--phényle, -$CH_2$-S-phényle, -$CH_2$-SO-phényle ou -$CH_2$-$SO_2$-phényle substitués chacun une à deux fois, de manière identique ou différente, en citant comme substituants du phényle les significations de Y indiquées ci-après,

ou bien

$R^1$ représente un -O-phényle, -S-phényle, -SO--phényle ou -$SO_2$-phényle substitués une à deux fois, de manière identique ou différente, en citant comme substituants du phényle les significations de Y indiquées ci-après,

$R^2$ un alcoyle à chaîne droite ou ramifiée ayant 1 à 4 atomes de carbone,

$R^3$ un alcoyle à chaîne droite ou ramifiée ayant 1 à 4 atomes de carbone,

X un atome d'azote ou le groupe CH,

Y de l'halogène, alcoyle ayant 1 à 4 atomes de carbone, cycloalcoyle ayant 5 à 7 atomes de carbone, alcoxy et alcoylthio ayant 1 à 4 atomes de carbone, halogénoalcoyle et halogénoalcoxy de même que halogénoalcoylthio ayant chacun 1 à 2 atomes de carbone et 1 à 5 atomes d'halogène identiques ou différents, de même qu'un phényle, phénoxy éventuellement substitués chacun par de l'halogène et par un alcoyle ayant 1 à 2 atomes de carbone, phénylalcoyle de même que phénylalcoxy ayant 1 à 2 atomes de carbone dans la portion alcoyle ou dans la portion alcoxy, et

m représente les nombres 0, 1, 2 ou 3,

ou de leurs sels d'addition d'acides et complexes avec des sels métalliques pour combattre les champignons chez les plantes.

2. Utilisation selon la revendication 1, caractérisée en ce que dans la formule (I)

$R^1$ représente un phényle, -$CH_2$-phényle, -$CH_2$-O--phényle, -$CH_2$-S-phényle, -$CH_2$-SO-phényle ou -$CH_2$-$SO_2$-phényle éventuellement substitués chacun une à deux fois, de manière identique ou différente, en citant comme substituants du phényle les significations de Y indiquées ci-après,

$R^2$ un méthyle ou éthyle,

$R^3$ un méthyle ou éthyle,

X un atome d'azote ou le groupe CH,

Y du fluor, chlore, brome, méthyle, isopropyle, t-butyle, cyclohexyle, méthoxy, méthylthio, trifluorométhyle, trifluorométhoxy, trifluorométhylthio de même qu'un phényle, phénoxy, benzyle ou benzyloxy éventuellement substitués par du fluor, du chlore ou un méthyle et

m représente les nombres 0, 1 ou 2.

3. Utilisation selon la revendication 1, caractérisée en ce que dans la formule (I)

$R^1$ représente un -O-phényle, -S-phényle, -SO--phényle ou -$SO_2$-phényle chacun substitués une à deux fois, de manière identique ou différente, en citant comme substituants du phényle les significations de Y mentionnées ci-dessous,

$R^2$ un méthyle ou éthyle,

$R^3$ un méthyle ou éthyle,

X un atome d'azote ou le groupe CH,

Y du fluor, chlore, brome, méthyle, isopropyle, t-butyle, cyclohexyle, méthoxy, méthylthio, trifluorométhyle, trifluorométhoxy, trifluorométhylthio, de même qu'un phényle, phénoxy, benzyle ou benzyloxy éventuellement substitués par du fluor, du chlore, un méthyle, et

m représente les nombres 0, 1 ou 2.